# EUROPEAN PATENT APPLICATION

(11) **EP 4 749 644 A1**
(43) Date of publication of application: **27.05.2026**
(21) Application number: 25217377.8
(22) Date of filing: 20.11.2025
(51) Int. Cl.: G16H 20/40, G16H 40/63

(54) **VISUALIZATION OF AUTOMATED SURGICAL SYSTEM DECISIONS**

(30) Priority: 20.11.2024 US 202418954199
(71) Applicant: Cilag GmbH International, 6300 Zug (CH)
(72) Inventor: SHELTON IV, Frederick E., Cincinnati, 45242 (US); BRADY, John E., Cincinnati, 45242 (US); JONES, Shannon L., Cincinnati, 45242 (US); HARRIS, Jason L., Cincinnati, 45242 (US); COWPERTHWAIT, Matthew David, Cincinnati, 45242 (US)
(74) Representative: Carpmaels & Ransford LLP

(57) **Abstract**

Device and methods for visualization of automated surgical system decisions. An example device may select a candidate action to perform based on steps of a surgical procedure and the effects of the candidate action. The effects may include whether the candidate action will impair the ability of a surgical instrument to perform a later step in the procedure. The candidate action may involve initial device placement, device movements, port placement, and/or the like.

## Description

### CROSS-REFERENCE TO RELATED APPLICATIONS

This application claims the benefit of the following, the disclosures of which are incorporated herein by reference in its entirety:
- Provisional U.S. Patent Application No. 63/602,040, filed November 22, 2023,
- Provisional U.S. Patent Application No. 63/602,028, filed November 22, 2023,
- Provisional U.S. Patent Application No. 63/601,998, filed November 22, 2023,
- Provisional U.S. Patent Application No. 63/602,003, filed November 22, 2023,
- Provisional U.S. Patent Application No. 63/602,006, filed November 22, 2023,
- Provisional U.S. Patent Application No. 63/602,011, filed November 22, 2023,
- Provisional U.S. Patent Application No. 63/602,013, filed November 22, 2023,
- Provisional U.S. Patent Application No. 63/602,037, filed November 22, 2023,
- Provisional U.S. Patent Application No. 63/602,007, filed November 22, 2023,
- Provisional U.S. Patent Application No. 63/603,031, filed November 27, 2023, and
- Provisional U.S. Patent Application No. 63/603,033, filed November 27, 2023.

This application is related to the following, filed contemporaneously, the contents of each of which are incorporated by reference herein:
- U.S. Patent Application No. 18/954,186, filed November 20, 2024, entitled METHOD FOR MULTI-SYSTEM INTERACTION,
- U.S. Patent Application No. 18/954,195, filed November 20, 2024, entitled, VISUALIZATION OF AN INTERNAL PROCESS OF AN AUTOMATED OPERATION,
- U.S. Patent Application No. 18/954,205, filed November 20, 2024, entitled VISUALIZATION OF EFFECTS OF DEVICE PLACEMENT IN AN OPERATING ROOM,
- U.S. Patent Application No. 18/954,178, filed November 20, 2024, entitled VISUALIZATION OF EFFECTS OF DEVICE MOVEMENTS IN AN OPERATING ROOM, and
- U.S. Patent Application No. 18/954,214, filed November 20, 2024, entitled DISPLAY OF COMPLEX AND CONFLICTING INTERRELATED DATA STREAMS.

### BACKGROUND

Surgical procedures are typically performed in surgical operating theaters or rooms in a healthcare facility such as, for example, a hospital. Various surgical devices and systems are utilized in performance of a surgical procedure. In the digital and information age, medical systems and facilities are often slower to implement systems or procedures utilizing newer and improved technologies due to patient safety and a general desire for maintaining traditional practices.

### SUMMARY

Devices and methods for visualizing automated surgical system decisions. An example device may include a processor configured to perform one or more actions. The device may receive an indication of a surgical procedure that involves a first surgical instrument cooperating with a second surgical instrument. The surgical procedure may include a plurality of surgical steps. The device may determine a first candidate action and a second candidate action associated with the first surgical instrument. The first candidate action and the second candidate action may allow the first surgical instrument to complete a first step of the plurality of the surgical steps. The device may determine a first effect, caused by the first candidate action, on the second surgical instrument's ability to perform a second step of the plurality of surgical steps. The device may determine a second effect, caused by the second candidate action, on the second surgical instrument's ability to perform the second step of the plurality of surgical steps. The device may select, based on the first effect and the second effect, an action, from the first candidate action and the second candidate action, for the first surgical instrument to perform. The device may generate a control signal configured to indicate the selected action.

The selected action may be a first action. The control signal may be a first control signal. The surgical procedure may include a third step. The device may determine, based on the selected first action associated with the first surgical instrument, a third candidate action and a fourth candidate action associated with the second surgical instrument. The third candidate action and the fourth candidate action may allow the second surgical instrument to complete the second step. The device may determine a third effect, caused by the selected candidate action associated with the first surgical instrument and the third candidate action, on a third surgical instrument's ability to perform the third step. The device may determine a fourth effect, caused by the selected candidate action associated with the first surgical instrument and the fourth candidate action, on the third surgical instrument's ability to perform the third step. The device may select, based on the third effect and the fourth effect, a second action, from the third candidate action and the fourth candidate action, for the second surgical instrument to perform. The device may generate a second control signal configured to indicate the second action.

The device may determine a parameter change associated with the first effect and the second effect. The device may determine a data type associated with the parameter change. The device may determine a format of a graphical representation of the first effect and the second effect based on the data type. The device may generate the graphical representation based on the determined format. The control signal may be configured to instruct a display to display the generated graphical representation.

The device may determine the data type associated with the parameter change based on one or more of: an absolute change in a parameter over a period of time; a relative change in the parameter over the period of time; data trends of summed data streams of interrelated parameters; or an impact, of the first or second effect, to one or more surgical instruments.

The device may identify a reserved space occupied by a third surgical instrument. Determining the first candidate action and the second candidate action associated with a first surgical instrument may involve determining that the first candidate action and the second candidate action cause the first surgical instrument to remain outside of the reserved space.

The first candidate action may involve placing a port in a first port location on a patient. The second candidate action may involve placing the port in a second port location on the patient. The control signal may be configured to indicate one or more of: a first magnitude of access that a laparoscopic instrument will have to a surgical area if the first port location is used, a first number of orientation possibilities of the laparoscopic instrument if the first port location is used, a second magnitude of access that the laparoscopic instrument will have to a surgical area if the second port location is used, or a second number of orientation possibilities of the laparoscopic instrument if the second port location is used.

The first surgical instrument may include a joint. The first candidate action may involve placing a port in a first port location on a patient. The second candidate action may involve placing the port in a second port location on the patient. The device may determine a first effect associated with the first port location and a second effect associated with the second port location based on at least one of: a position of a health care provider relative to the first surgical instrument, an articulation angle of the joint, a joint length of the joint, or a degree of freedom of the joint.

The device may receive user preference information and a patient position associated with the surgical procedure. The device may determine a surgical constraint based on at least one of the user preference information or the patient position. Selecting the action, from the first candidate action and the second candidate action, for the first surgical instrument to perform, may be based on the surgical constraint.

The first candidate action may involve a first placement of a base associated with the first surgical instrument, or a first movement of the first surgical instrument. The second candidate action may involve a second placement of the base associated with the first surgical instrument, or a second movement of the first surgical instrument.

### BRIEF DESCRIPTION OF THE DRAWINGS

FIG. 1 is a block diagram of a computer-implemented surgical system.
FIG. 2 illustrates an example surgical system in a surgical operating room.
FIG. 3 illustrates an example surgical hub paired with various systems.
FIG. 4 illustrates an example situationally aware surgical system.
FIG. 5 illustrates example joint movements of a robotic arm.
FIG. 6 illustrates an example operating room arrangement of multiple robotic arms.
FIG. 7 illustrates an example display to a user of potential interactions between robotic arms during a surgical procedure.
FIG. 8 illustrates an example display to a user of a decision to reposition a robotic arm to avoid a collision.
FIG. 9 illustrates an example display to a user of optional actions to avoid a collision.
FIG. 10 illustrates an example display to a user of the impact of potential port locations.
FIG. 11 illustrates an example display to a user of the access capabilities of surgical tools based on a port location.

### DETAILED DESCRIPTION

A more detailed understanding may be had from the following description, given by way of example in conjunction with the accompanying drawings.

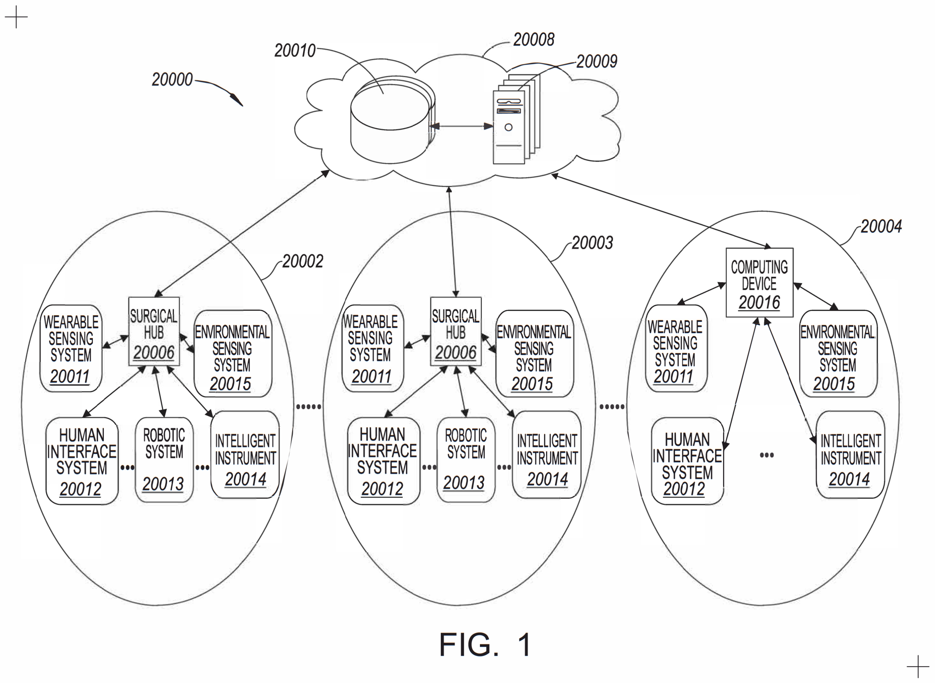

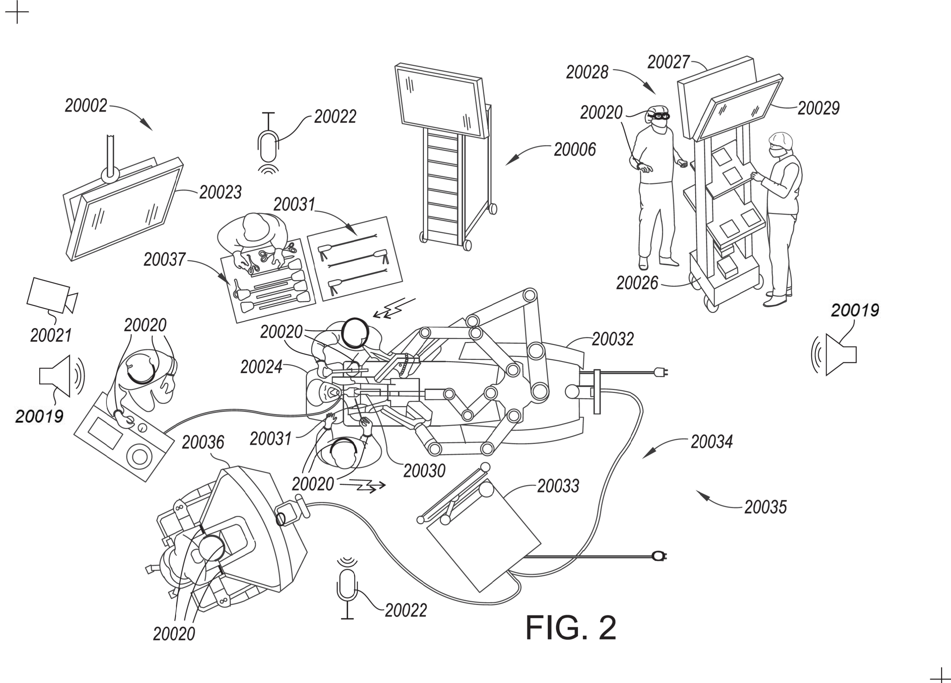

FIG. 1 shows an example computer-implemented surgical system 20000. The example surgical system 20000 may include one or more surgical systems (e.g., surgical sub-systems) 20002, 20003 and 20004. For example, surgical system 20002 may include a computer-implemented interactive surgical system. For example, surgical system 20002 may include a surgical hub 20006 and/or a computing device 20016 in communication with a cloud computing system 20008, for example, as described in FIG. 2. The cloud computing system 20008 may include at least one remote cloud server 20009 and at least one remote cloud storage unit 20010. Example surgical systems 20002, 20003, or 20004 may include one or more wearable sensing systems 20011, one or more environmental sensing systems 20015, one or more robotic systems 20013, one or more intelligent instruments 20014, one or more human interface systems 20012, etc. The human interface system is also referred herein as the human interface device. The wearable sensing system 20011 may include one or more health care professional (HCP) sensing systems, and/or one or more patient sensing systems. The environmental sensing system 20015 may include one or more devices, for example, used for measuring one or more environmental attributes, for example, as further described in FIG. 2. The robotic system 20013 may include a plurality of devices used for performing a surgical procedure, for example, as further described in FIG. 2.

The surgical system 20002 may be in communication with a remote server 20009 that may be part of a cloud computing system 20008. In an example, the surgical system 20002 may be in communication with a remote server 20009 via an internet service provider's cable/FIOS networking node. In an example, a patient sensing system may be in direct communication with a remote server 20009. The surgical system 20002 (and/or various sub-systems, smart surgical instruments, robots, sensing systems, and other computerized devices described herein) may collect data in real-time and transfer the data to cloud computers for data processing and manipulation. It will be appreciated that cloud computing may rely on sharing computing resources rather than having local servers or personal devices to handle software applications.

The surgical system 20002 and/or a component therein may communicate with the remote servers 20009 via a cellular transmission/reception point (TRP) or a base station using one or more of the following cellular protocols: GSM/GPRS/EDGE (2G), UMTS/HSPA (3G), long term evolution (LTE) or 4G, LTE-Advanced (LTE-A), new radio (NR) or 5G, and/or other wired or wireless communication protocols. Various examples of cloud-based analytics that are performed by the cloud computing system 20008, and are suitable for use with the present disclosure, are described in U.S. Patent Application Publication No. US 2019-0206569 A1 (U.S. Patent Application No. 16/209,403), titled METHOD OF CLOUD BASED DATA ANALYTICS FOR USE WITH THE HUB, filed December 4, 2018, the disclosure of which is herein incorporated by reference in its entirety.

The surgical hub 20006 may have cooperative interactions with one of more means of displaying the image from the laparoscopic scope and information from one or more other smart devices and one or more sensing systems 20011. The surgical hub 20006 may interact with one or more sensing systems 20011, one or more smart devices, and multiple displays. The surgical hub 20006 may be configured to gather measurement data from the sensing system(s) and send notifications or control messages to the one or more sensing systems 20011. The surgical hub 20006 may send and/or receive information including notification information to and/or from the human interface system 20012. The human interface system 20012 may include one or more human interface devices (HIDs). The surgical hub 20006 may send and/or receive notification information or control information to audio, display and/or control information to various devices that are in communication with the surgical hub.

For example, the sensing systems may include the wearable sensing system 20011 (which may include one or more HCP sensing systems and/or one or more patient sensing systems) and/or the environmental sensing system 20015 shown in FIG. 1. The sensing system(s) may measure data relating to various biomarkers. The sensing system(s) may measure the biomarkers using one or more sensors, for example, photosensors (e.g., photodiodes, photoresistors), mechanical sensors (e.g., motion sensors), acoustic sensors, electrical sensors, electrochemical sensors, thermoelectric sensors, infrared sensors, etc. The sensor(s) may measure the biomarkers as described herein using one of more of the following sensing technologies: photoplethysmography, electrocardiography, electroencephalography, colorimetry, impedimentary, potentiometry, amperometry, etc.

The biomarkers measured by the sensing systems may include, but are not limited to, sleep, core body temperature, maximal oxygen consumption, physical activity, alcohol consumption, respiration rate, oxygen saturation, blood pressure, blood sugar, heart rate variability, blood potential of hydrogen, hydration state, heart rate, skin conductance, peripheral temperature, tissue perfusion pressure, coughing and sneezing, gastrointestinal motility, gastrointestinal tract imaging, respiratory tract bacteria, edema, mental aspects, sweat, circulating tumor cells, autonomic tone, circadian rhythm, and/or menstrual cycle.

The biomarkers may relate to physiologic systems, which may include, but are not limited to, behavior and psychology, cardiovascular system, renal system, skin system, nervous system, gastrointestinal system, respiratory system, endocrine system, immune system, tumor, musculoskeletal system, and/or reproductive system. Information from the biomarkers may be determined and/or used by the computer-implemented patient and the surgical system 20000, for example. The information from the biomarkers may be determined and/or used by the computer-implemented patient and the surgical system 20000 to improve said systems and/or to improve patient outcomes, for example.

The sensing systems may send data to the surgical hub 20006. The sensing systems may use one or more of the following RF protocols for communicating with the surgical hub 20006: Bluetooth, Bluetooth Low-Energy (BLE), Bluetooth Smart, Zigbee, Z-wave, IPv6 Low-power wireless Personal Area Network (6LoWPAN), Wi-Fi.

The sensing systems, biomarkers, and physiological systems are described in more detail in U.S. App No. 17/156,287 (attorney docket number END9290USNP1), titled METHOD OF ADJUSTING A SURGICAL PARAMETER BASED ON BIOMARKER MEASUREMENTS, filed January 22, 2021, the disclosure of which is herein incorporated by reference in its entirety.

The sensing systems described herein may be employed to assess physiological conditions of a surgeon operating on a patient or a patient being prepared for a surgical procedure or a patient recovering after a surgical procedure. The cloud-based computing system 20008 may be used to monitor biomarkers associated with a surgeon or a patient in real-time and to generate surgical plans based at least on measurement data gathered prior to a surgical procedure, provide control signals to the surgical instruments during a surgical procedure, and notify a patient of a complication during post-surgical period.

The cloud-based computing system 20008 may be used to analyze surgical data. Surgical data may be obtained via one or more intelligent instrument(s) 20014, wearable sensing system(s) 20011, environmental sensing system(s) 20015, robotic system(s) 20013 and/or the like in the surgical system 20002. Surgical data may include tissue states to assess leaks or perfusion of sealed tissue after a tissue sealing and cutting procedure pathology data, including images of samples of body tissue, anatomical structures of the body using a variety of sensors integrated with imaging devices and techniques such as overlaying images captured by multiple imaging devices, image data, and/or the like. The surgical data may be analyzed to improve surgical procedure outcomes by determining if further treatment, such as the application of endoscopic intervention, emerging technologies, a targeted radiation, targeted intervention, and precise robotics to tissue-specific sites and conditions. Such data analysis may employ outcome analytics processing and using standardized approaches may provide beneficial feedback to either confirm surgical treatments and the behavior of the surgeon or suggest modifications to surgical treatments and the behavior of the surgeon.

FIG. 2 shows an example surgical system 20002 in a surgical operating room. As illustrated in FIG. 2, a patient is being operated on by one or more health care professionals (HCPs). The HCPs are being monitored by one or more HCP sensing systems 20020 worn by the HCPs. The HCPs and the environment surrounding the HCPs may also be monitored by one or more environmental sensing systems including, for example, a set of cameras 20021, a set of microphones 20022, and other sensors that may be deployed in the operating room. The HCP sensing systems 20020 and the environmental sensing systems may be in communication with a surgical hub 20006, which in turn may be in communication with one or more cloud servers 20009 of the cloud computing system 20008, as shown in FIG. 1. The environmental sensing systems may be used for measuring one or more environmental attributes, for example, HCP position in the surgical theater, HCP movements, ambient noise in the surgical theater, temperature/humidity in the surgical theater, etc.

As illustrated in FIG. 2, a primary display 20023 and one or more audio output devices (e.g., speakers 20019) are positioned in the sterile field to be visible to an operator at the operating table 20024. In addition, a visualization/notification tower 20026 is positioned outside the sterile field. The visualization/notification tower 20026 may include a first non-sterile human interactive device (HID) 20027 and a second non-sterile HID 20029, which may face away from each other. The HID may be a display or a display with a touchscreen allowing a human to interface directly with the HID. A human interface system, guided by the surgical hub 20006, may be configured to utilize the HIDs 20027, 20029, and 20023 to coordinate information flow to operators inside and outside the sterile field. In an example, the surgical hub 20006 may cause an HID (e.g., the primary HID 20023) to display a notification and/or information about the patient and/or a surgical procedure step. In an example, the surgical hub 20006 may prompt for and/or receive input from personnel in the sterile field or in the non-sterile area. In an example, the surgical hub 20006 may cause an HID to display a snapshot of a surgical site, as recorded by an imaging device 20030, on a non-sterile HID 20027 or 20029, while maintaining a live feed of the surgical site on the primary HID 20023. The snapshot on the non-sterile display 20027 or 20029 can permit a non-sterile operator to perform a diagnostic step relevant to the surgical procedure, for example.

The surgical hub 20006 may be configured to route a diagnostic input or feedback entered by a non-sterile operator at the visualization tower 20026 to the primary display 20023 within the sterile field, where it can be viewed by a sterile operator at the operating table. In an example, the input can be in the form of a modification to the snapshot displayed on the non-sterile display 20027 or 20029, which can be routed to the primary display 20023 by the surgical hub 20006.

Referring to FIG. 2, a surgical instrument 20031 is being used in the surgical procedure as part of the surgical system 20002. The hub 20006 may be configured to coordinate information flow to a display of the surgical instrument(s) 20031. For example, in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety. A diagnostic input or feedback entered by a non-sterile operator at the visualization tower 20026 can be routed by the hub 20006 to the surgical instrument display within the sterile field, where it can be viewed by the operator of the surgical instrument 20031. Example surgical instruments that are suitable for use with the surgical system 20002 are described under the heading "Surgical Instrument Hardware" and in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety, for example.

As shown in FIG. 2, the surgical system 20002 can be used to perform a surgical procedure on a patient who is lying down on an operating table 20024 in a surgical operating room 20035. A robotic system 20034 may be used in the surgical procedure as a part of the surgical system 20002. The robotic system 20034 may include a surgeon's console 20036, a patient side cart 20032 (surgical robot), and a surgical robotic hub 20033. The patient side cart 20032 can manipulate at least one removably coupled surgical tool 20037 through a minimally invasive incision in the body of the patient while the surgeon views the surgical site through the surgeon's console 20036. An image of the surgical site can be obtained by a medical imaging device 20030, which can be manipulated by the patient side cart 20032 to orient the imaging device 20030. The robotic hub 20033 can be used to process the images of the surgical site for subsequent display to the surgeon through the surgeon's console 20036.

Other types of robotic systems can be readily adapted for use with the surgical system 20002. Various examples of robotic systems and surgical tools that are suitable for use with the present disclosure are described herein, as well as in U.S. Patent Application Publication No. US 2019-0201137 A1 (U.S. Patent Application No. 16/209,407), titled METHOD OF ROBOTIC HUB COMMUNICATION, DETECTION, AND CONTROL, filed December 4, 2018, the disclosure of which is herein incorporated by reference in its entirety.

In various aspects, the imaging device 20030 may include at least one image sensor and one or more optical components. Suitable image sensors may include, but are not limited to, Charge-Coupled Device (CCD) sensors and Complementary Metal-Oxide Semiconductor (CMOS) sensors.

The optical components of the imaging device 20030 may include one or more illumination sources and/or one or more lenses. The one or more illumination sources may be directed to illuminate portions of the surgical field. The one or more image sensors may receive light reflected or refracted from the surgical field, including light reflected or refracted from tissue and/or surgical instruments.

The illumination source(s) may be configured to radiate electromagnetic energy in the visible spectrum as well as the invisible spectrum. The visible spectrum, sometimes referred to as the optical spectrum or luminous spectrum, is the portion of the electromagnetic spectrum that is visible to (e.g., can be detected by) the human eye and may be referred to as visible light or simply light. A typical human eye will respond to wavelengths in air that range from about 380 nm to about 750 nm.

The invisible spectrum (e.g., the non-luminous spectrum) is the portion of the electromagnetic spectrum that lies below and above the visible spectrum (i.e., wavelengths below about 380 nm and above about 750 nm). The invisible spectrum is not detectable by the human eye. Wavelengths greater than about 750 nm are longer than the red visible spectrum, and they become invisible infrared (IR), microwave, and radio electromagnetic radiation. Wavelengths less than about 380 nm are shorter than the violet spectrum, and they become invisible ultraviolet, x-ray, and gamma ray electromagnetic radiation.

In various aspects, the imaging device 20030 is configured for use in a minimally invasive procedure. Examples of imaging devices suitable for use with the present disclosure include, but are not limited to, an arthroscope, angioscope, bronchoscope, choledochoscope, colonoscope, cytoscope, duodenoscope, enteroscope, esophagogastro-duodenoscope (gastroscope), endoscope, laryngoscope, nasopharyngo-neproscope, sigmoidoscope, thoracoscope, and ureteroscope.

The imaging device may employ multi-spectrum monitoring to discriminate topography and underlying structures. A multi-spectral image is one that captures image data within specific wavelength ranges across the electromagnetic spectrum. The wavelengths may be separated by filters or by the use of instruments that are sensitive to particular wavelengths, including light from frequencies beyond the visible light range, e.g., IR and ultraviolet. Spectral imaging can allow extraction of additional information that the human eye fails to capture with its receptors for red, green, and blue. The use of multi-spectral imaging is described in greater detail under the heading "Advanced Imaging Acquisition Module" in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety. Multi-spectrum monitoring can be a useful tool in relocating a surgical field after a surgical task is completed to perform one or more of the previously described tests on the treated tissue. It is axiomatic that strict sterilization of the operating room and surgical equipment is required during any surgery. The strict hygiene and sterilization conditions required in a "surgical theater," e.g., an operating or treatment room, necessitate the highest possible sterility of all medical devices and equipment. Part of that sterilization process is the need to sterilize anything that comes in contact with the patient or penetrates the sterile field, including the imaging device 20030 and its attachments and components. It will be appreciated that the sterile field may be considered a specified area, such as within a tray or on a sterile towel, that is considered free of microorganisms, or the sterile field may be considered an area, immediately around a patient, who has been prepared for a surgical procedure. The sterile field may include the scrubbed team members, who are properly attired, and all furniture and fixtures in the area.

Wearable sensing system 20011 illustrated in FIG. 1 may include one or more HCP sensing systems 20020 as shown in FIG. 2. The HCP sensing systems 20020 may include sensing systems to monitor and detect a set of physical states and/or a set of physiological states of a healthcare personnel (HCP). An HCP may be a surgeon or one or more healthcare personnel assisting the surgeon or other healthcare service providers in general. In an example, an HCP sensing system 20020 may measure a set of biomarkers to monitor the heart rate of an HCP. In an example, an HCP sensing system 20020 worn on a surgeon's wrist (e.g., a watch or a wristband) may use an accelerometer to detect hand motion and/or shakes and determine the magnitude and frequency of tremors. The sensing system 20020 may send the measurement data associated with the set of biomarkers and the data associated with a physical state of the surgeon to the surgical hub 20006 for further processing.

The environmental sensing system(s) 20015 shown in FIG. 1 may send environmental information to the surgical hub 20006. For example, the environmental sensing system(s) 20015 may include a camera 20021 for detecting hand/body position of an HCP. The environmental sensing system(s) 20015 may include microphones 20022 for measuring the ambient noise in the surgical theater. Other environmental sensing system(s) 20015 may include devices, for example, a thermometer to measure temperature and a hygrometer to measure humidity of the surroundings in the surgical theater, etc. The surgeon biomarkers may include one or more of the following: stress, heart rate, etc. The environmental measurements from the surgical theater may include ambient noise level associated with the surgeon or the patient, surgeon and/or staff movements, surgeon and/or staff attention level, etc. The surgical hub 20006, alone or in communication with the cloud computing system, may use the surgeon biomarker measurement data and/or environmental sensing information to modify the control algorithms of hand-held instruments or the averaging delay of a robotic interface, for example, to minimize tremors.

The surgical hub 20006 may use the surgeon biomarker measurement data associated with an HCP to adaptively control one or more surgical instruments 20031. For example, the surgical hub 20006 may send a control program to a surgical instrument 20031 to control its actuators to limit or compensate for fatigue and use of fine motor skills. The surgical hub 20006 may send the control program based on situational awareness and/or the context on importance or criticality of a task. The control program may instruct the instrument to alter operation to provide more control when control is needed.

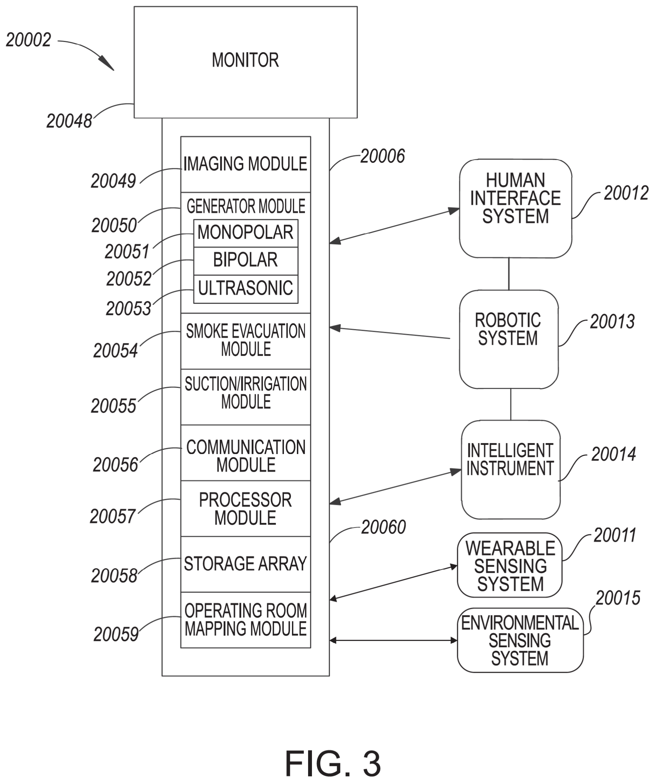

FIG. 3 shows an example surgical system 20002 with a surgical hub 20006. The surgical hub 20006 may be paired with, via a modular control, a wearable sensing system 20011, an environmental sensing system 20015, a human interface system 20012, a robotic system 20013, and an intelligent instrument 20014. The hub 20006 includes a display 20048, an imaging module 20049, a generator module 20050 (e.g., an energy generator), a communication module 20056, a processor module 20057, a storage array 20058, and an operating-room mapping module 20059. In certain aspects, as illustrated in FIG. 3, the hub 20006 further includes a smoke evacuation module 20054 and/or a suction/irrigation module 20055. The various modules and systems may be connected to the modular control either directly via a router or via the communication module 20056. The operating theater devices may be coupled to cloud computing resources and data storage via the modular control. The human interface system 20012 may include a display sub-system and a notification sub-system.

The modular control may be coupled to non-contact sensor module. The non-contact sensor module may measure the dimensions of the operating theater and generate a map of the surgical theater using ultrasonic, laser-type, and/or the like, non-contact measurement devices. Other distance sensors can be employed to determine the bounds of an operating room. An ultrasound-based non-contact sensor module may scan the operating theater by transmitting a burst of ultrasound and receiving the echo when it bounces off the perimeter walls of an operating theater as described under the heading "Surgical Hub Spatial Awareness Within an Operating Room" in U.S. Provisional Patent Application Serial No. 62/611,341, titled INTERACTIVE SURGICAL PLATFORM, filed December 28, 2017, which is herein incorporated by reference in its entirety. The sensor module may be configured to determine the size of the operating theater and to adjust Bluetooth-pairing distance limits. A laser-based non-contact sensor module may scan the operating theater by transmitting laser light pulses, receiving laser light pulses that bounce off the perimeter walls of the operating theater, and comparing the phase of the transmitted pulse to the received pulse to determine the size of the operating theater and to adjust Bluetooth pairing distance limits, for example.

During a surgical procedure, energy application to tissue, for sealing and/or cutting, may be associated with smoke evacuation, suction of excess fluid, and/or irrigation of the tissue. Fluid, power, and/or data lines from different sources may be entangled during the surgical procedure. Valuable time can be lost addressing this issue during a surgical procedure. Detangling the lines may necessitate disconnecting the lines from their respective modules, which may require resetting the modules. The hub modular enclosure 20060 may offer a unified environment for managing the power, data, and fluid lines, which reduces the frequency of entanglement between such lines.

Energy may be applied to tissue at a surgical site. The surgical hub 20006 may include a hub enclosure 20060 and a combo generator module slidably receivable in a docking station of the hub enclosure 20060. The docking station may include data and power contacts. The combo generator module may include two or more of: an ultrasonic energy generator component, a bipolar RF energy generator component, or a monopolar RF energy generator component that are housed in a single unit. The combo generator module may include a smoke evacuation component, at least one energy delivery cable for connecting the combo generator module to a surgical instrument, at least one smoke evacuation component configured to evacuate smoke, fluid, and/or particulates generated by the application of therapeutic energy to the tissue, and a fluid line extending from the remote surgical site to the smoke evacuation component. The fluid line may be a first fluid line, and a second fluid line may extend from the remote surgical site to a suction and irrigation module 20055 slidably received in the hub enclosure 20060. The hub enclosure 20060 may include a fluid interface.

The combo generator module may generate multiple energy types for application to the tissue. One energy type may be more beneficial for cutting the tissue, while another different energy type may be more beneficial for sealing the tissue. For example, a bipolar generator can be used to seal the tissue while an ultrasonic generator can be used to cut the sealed tissue. Aspects of the present disclosure present a solution where a hub modular enclosure 20060 is configured to accommodate different generators and facilitate an interactive communication therebetween. The hub modular enclosure 20060 may enable the quick removal and/or replacement of various modules.

The modular surgical enclosure may include a first energy-generator module, configured to generate a first energy for application to the tissue, and a first docking station comprising a first docking port that includes first data and power contacts, wherein the first energy-generator module is slidably movable into an electrical engagement with the power and data contacts and wherein the first energy-generator module is slidably movable out of the electrical engagement with the first power and data contacts. The modular surgical enclosure may include a second energy-generator module configured to generate a second energy, different than the first energy, for application to the tissue, and a second docking station comprising a second docking port that includes second data and power contacts, wherein the second energy generator module is slidably movable into an electrical engagement with the power and data contacts, and wherein the second energy-generator module is slidably movable out of the electrical engagement with the second power and data contacts. In addition, the modular surgical enclosure also includes a communication bus between the first docking port and the second docking port, configured to facilitate communication between the first energy-generator module and the second energy-generator module.

Referring to FIG. 3, the hub modular enclosure 20060 may allow the modular integration of a generator module 20050, a smoke evacuation module 20054, and a suction/irrigation module 20055. The hub modular enclosure 20060 may facilitate interactive communication between the modules 20059, 20054, and 20055. The generator module 20050 can be with integrated monopolar, bipolar, and ultrasonic components supported in a single housing unit slidably insertable into the hub modular enclosure 20060. The generator module 20050 may connect to a monopolar device 20051, a bipolar device 20052, and an ultrasonic device 20053. The generator module 20050 may include a series of monopolar, bipolar, and/or ultrasonic generator modules that interact through the hub modular enclosure 20060. The hub modular enclosure 20060 may facilitate the insertion of multiple generators and interactive communication between the generators docked into the hub modular enclosure 20060 so that the generators would act as a single generator.

A surgical data network having a set of communication hubs may connect the sensing system(s), the modular devices located in one or more operating theaters of a healthcare facility, a patient recovery room, or a room in a healthcare facility specially equipped for surgical operations, to the cloud computing system 20008.

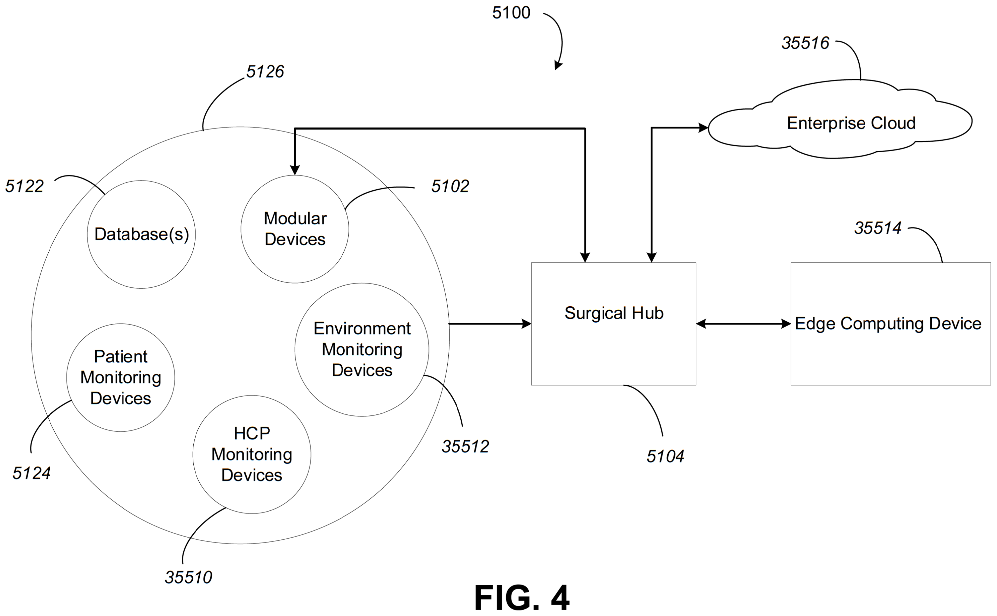

FIG. 4 illustrates a diagram of a situationally aware surgical system 5100. The data sources 5126 may include, for example, the modular devices 5102, databases 5122 (e.g., an EMR database containing patient records), patient monitoring devices 5124 (e.g., a blood pressure (BP) monitor and an electrocardiography (EKG) monitor), HCP monitoring devices 35510, and/or environment monitoring devices 35512. The modular devices 5102 may include sensors configured to detect parameters associated with the patient, HCPs and environment and/or the modular device itself. The modular devices 5102 may include one or more intelligent instrument(s) 20014. The surgical hub 5104 may derive the contextual information pertaining to the surgical procedure from the data based upon, for example, the particular combination(s) of received data or the particular order in which the data is received from the data sources 5126. The contextual information inferred from the received data can include, for example, the type of surgical procedure being performed, the particular step of the surgical procedure that the surgeon is performing, the type of tissue being operated on, or the body cavity that is the subject of the procedure. This ability by some aspects of the surgical hub 5104 to derive or infer information related to the surgical procedure from received data can be referred to as "situational awareness." For example, the surgical hub 5104 can incorporate a situational awareness system, which may be the hardware and/or programming associated with the surgical hub 5104 that derives contextual information pertaining to the surgical procedure from the received data and/or a surgical plan information received from the edge computing system 35514 or an enterprise cloud server 35516. The contextual information derived from the data sources 5126 may include, for example, what step of the surgical procedure is being performed, whether and how a particular modular device 5102 is being used, and the patient's condition.

The surgical hub 5104 may be connected to various databases 5122 to retrieve therefrom data regarding the surgical procedure that is being performed or is to be performed. In one exemplification of the surgical system 5100, the databases 5122 may include an EMR database of a hospital. The data that may be received by the situational awareness system of the surgical hub 5104 from the databases 5122 may include, for example, start (or setup) time or operational information regarding the procedure (e.g., a segmentectomy in the upper right portion of the thoracic cavity). The surgical hub 5104 may derive contextual information regarding the surgical procedure from this data alone or from the combination of this data and data from other data sources 5126.

The surgical hub 5104 may be connected to (e.g., paired with) a variety of patient monitoring devices 5124. In an example of the surgical system 5100, the patient monitoring devices 5124 that can be paired with the surgical hub 5104 may include a pulse oximeter (SpO2 monitor) 5114, a BP monitor 5116, and an EKG monitor 5120. The perioperative data that is received by the situational awareness system of the surgical hub 5104 from the patient monitoring devices 5124 may include, for example, the patient's oxygen saturation, blood pressure, heart rate, and other physiological parameters. The contextual information that may be derived by the surgical hub 5104 from the perioperative data transmitted by the patient moni-toring devices 5124 may include, for example, whether the patient is located in the operating theater or under anesthesia. The surgical hub 5104 may derive these inferences from data from the patient monitoring devices 5124 alone or in combination with data from other data sources 5126 (e.g., the ventilator 5118).

The surgical hub 5104 may be connected to (e.g., paired with) a variety of modular devices 5102. In one exemplification of the surgical system 5100, the modular devices 5102 that are paired with the surgical hub 5104 may include a smoke evacuator, a medical imaging device such as the imaging device 20030 shown in FIG. 2, an insufflator, a combined energy generator (for powering an ultrasonic surgical instrument and/or an RF electrosurgical instrument), and a ventilator.

The perioperative data received by the surgical hub 5104 from the medical imaging device may include, for example, whether the medical imaging device is activated and a video or image feed. The contextual information that is derived by the surgical hub 5104 from the perioperative data sent by the medical imaging device may include, for example, whether the procedure is a VATS procedure (based on whether the medical imaging device is activated or paired to the surgical hub 5104 at the beginning or during the course of the procedure). The image or video data from the medical imaging device (or the data stream representing the video for a digital medical imaging device) may be processed by a pattern recognition system or a machine learning system to recognize features (e.g., organs or tissue types) in the field of view (FOY) of the medical imaging device, for example. The contextual information that is derived by the surgical hub 5104 from the recognized features may include, for example, what type of surgical procedure (or step thereof) is being performed, what organ is being operated on, or what body cavity is being operated in.

The situational awareness system of the surgical hub 5104 may derive the contextual information from the data received from the data sources 5126 in a variety of different ways. For example, the situational awareness system can include a pattern recognition system, or machine learning system (e.g., an artificial neural network), that has been trained on training data to correlate various inputs (e.g., data from database(s) 5122, patient monitoring devices 5124, modular devices 5102, HCP monitoring devices 35510, and/or environment monitoring devices 35512) to corresponding contextual information regarding a surgical procedure. For example, a machine learning system may accurately derive contextual information regarding a surgical procedure from the provided inputs. In examples, the situational awareness system can include a lookup table storing pre-characterized contextual information regarding a surgical procedure in association with one or more inputs (or ranges of inputs) corresponding to the contextual information. In response to a query with one or more inputs, the lookup table can return the corresponding contextual information for the situational awareness system for controlling the modular devices 5102. In examples, the contextual information received by the situational awareness system of the surgical hub 5104 can be associated with a particular control adjustment or set of control adjustments for one or more modular devices 5102. In examples, the situational awareness system can include a machine learning system, lookup table, or other such system, which may generate or retrieve one or more control adjustments for one or more modular devices 5102 when provided the contextual information as input.

For example, based on the data sources 5126, the situationally aware surgical hub 5104 may determine what type of tissue was being operated on. The situationally aware surgical hub 5104 can infer whether a surgical procedure being performed is a thoracic or an abdominal procedure, allowing the surgical hub 5104 to determine whether the tissue clamped by an end effector of the surgical stapling and cutting instrument is lung (for a thoracic procedure) or stomach (for an abdominal procedure) tissue. The situationally aware surgical hub 5104 may determine whether the surgical site is under pressure (by determining that the surgical procedure is utilizing insufflation) and determine the procedure type, for a consistent amount of smoke evacuation for both thoracic and abdominal procedures. Based on the data sources 5126, the situationally aware surgical hub 5104 could determine what step of the surgical procedure is being performed or will subsequently be performed.

The situationally aware surgical hub 5104 could determine what type of surgical procedure is being performed and customize the energy level according to the expected tissue profile for the surgical procedure. The situationally aware surgical hub 5104 may adjust the energy level for the ultrasonic surgical instrument or RF electrosurgical instrument throughout the course of a surgical procedure, rather than just on a procedure-by-procedure basis.

In examples, data can be drawn from additional data sources 5126 to improve the conclusions that the surgical hub 5104 draws from one data source 5126. The situationally aware surgical hub 5104 could augment data that it receives from the modular devices 5102 with contextual information that it has built up regarding the surgical procedure from other data sources 5126.

The situational awareness system of the surgical hub 5104 can consider the physiological measurement data to provide additional context in analyzing the visualization data. The additional context can be useful when the visualization data may be inconclusive or incomplete on its own.

The situationally aware surgical hub 5104 could determine whether the surgeon (or other HCP(s)) was making an error or otherwise deviating from the expected course of action during the course of a surgical procedure. For example, the surgical hub 5104 may determine the type of surgical procedure being performed, retrieve the corresponding list of steps or order of equipment usage (e.g., from a memory), and compare the steps being performed or the equipment being used during the course of the surgical procedure to the expected steps or equipment for the type of surgical procedure that the surgical hub 5104 determined is being performed. The surgical hub 5104 can provide an alert indicating that an unexpected action is being performed or an unexpected device is being utilized at the particular step in the surgical procedure.

The surgical instruments (and other modular devices 5102) may be adjusted for the particular context of each surgical procedure (such as adjusting to different tissue types) and validating actions during a surgical procedure. Next steps, data, and display adjustments may be provided to surgical instruments (and other modular devices 5102) in the surgical theater according to the specific context of the procedure.

A smart system may request (e.g., require) input from a healthcare provider (HCP). The smart system may reduce the number of displayable options (e.g., to a more manageable group from which the HCP can choose).

The system may display multiple HCP choices. The choices may include future actions and/or probabilities of success. The smart system may have operational functions that can interfere (e.g., adjust parameters). If the system is using a data stream from another smart system, the data stream may be used to define at least two options that would result in different operations. The system may display the options to the user. For example, the system may display future steps of use and/or a parameter that is related to the probability of the system operating as desired. There may be multiple levels of choices. For example, the levels may have (e.g., require) varying levels of inputs from the HCP (e.g., surgeon). The levels may request minimal input (e.g., no say) or detailed intervention.

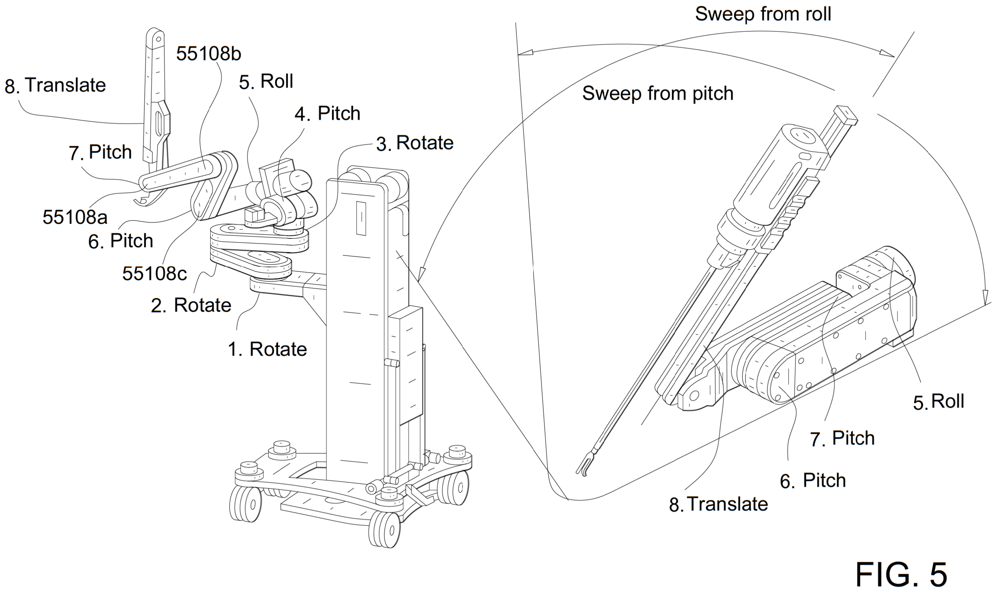

FIG. 5 illustrates example joint movements of a robotic arm attached to a base. As shown, the arm may be able to move in the x, y, and z directions. The joints may control the pitch, roll, and rotation of an end effector attached to the arm. The arm may have a mechanism (e.g., slide) for translation of the end effector. The joints may cause the end effector to make small movements inside the patient (e.g., in the surgical cavity). The arm movements correlated to the small internal movements may be large (e.g., creating a considerable sweep of the arm).

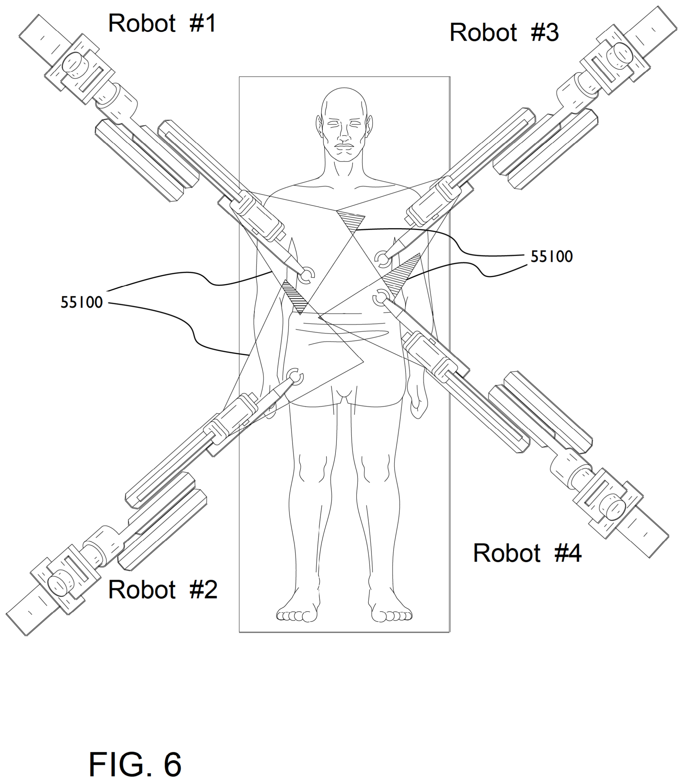

FIG. 6 illustrates an example operating room arrangement of multiple robotic arms. As shown, multiple robotic arms may be present in a relatively small space in an OR. The arms may have ranges of motion that overlap (e.g., as shown at 55100). In this case, the arms may collide unless adjustments are made to reduce the potential interactions. For example, one or more of the arms may not be allowed to occupy the overlapping space (e.g., at a given time). As described herein, the system may determine potential robot arm position placement and kinematic forecasting of resulting positions, movements, and/or interactions. The system may determine (and display) options for the HCP to choose from (e.g., and request that the HCP provide input). The system may output a simple, understandable display of context and choices.

Legibility (e.g., which is traditionally an attribute of written text) may refer to the quality of being easy to read and/or understand. A legible display of motion may involve (e.g., require) a conscious effort to make the diagramming clear and readable to a user (e.g., HCP).

Devices and methods for visualizing automated surgical system decisions. An example device may include a processor configured to perform one or more actions. The device may receive an indication of a surgical procedure that involves a first surgical instrument cooperating with a second surgical instrument. The surgical procedure may include a plurality of surgical steps. The device may determine a first candidate action and a second candidate action associated with the first surgical instrument. The first candidate action and the second candidate action may allow the first surgical instrument to complete a first step of the plurality of the surgical steps. The device may determine a first effect, caused by the first candidate action, on the second surgical instrument's ability to perform a second step of the plurality of surgical steps. The device may determine a second effect, caused by the second candidate action, on the second surgical instrument's ability to perform the second step of the plurality of surgical steps. The device may select, based on the first effect and the second effect, an action, from the first candidate action and the second candidate action, for the first surgical instrument to perform. The device may generate a control signal configured to indicate the selected action.

The selected action may be a first action. The control signal may be a first control signal. The surgical procedure may include a third step. The device may determine, based on the selected first action associated with the first surgical instrument, a third candidate action and a fourth candidate action associated with the second surgical instrument. The third candidate action and the fourth candidate action may allow the second surgical instrument to complete the second step. The device may determine a third effect, caused by the selected candidate action associated with the first surgical instrument and the third candidate action, on a third surgical instrument's ability to perform the third step. The device may determine a fourth effect, caused by the selected candidate action associated with the first surgical instrument and the fourth candidate action, on the third surgical instrument's ability to perform the third step. The device may select, based on the third effect and the fourth effect, a second action, from the third candidate action and the fourth candidate action, for the second surgical instrument to perform. The device may generate a second control signal configured to indicate the second action.

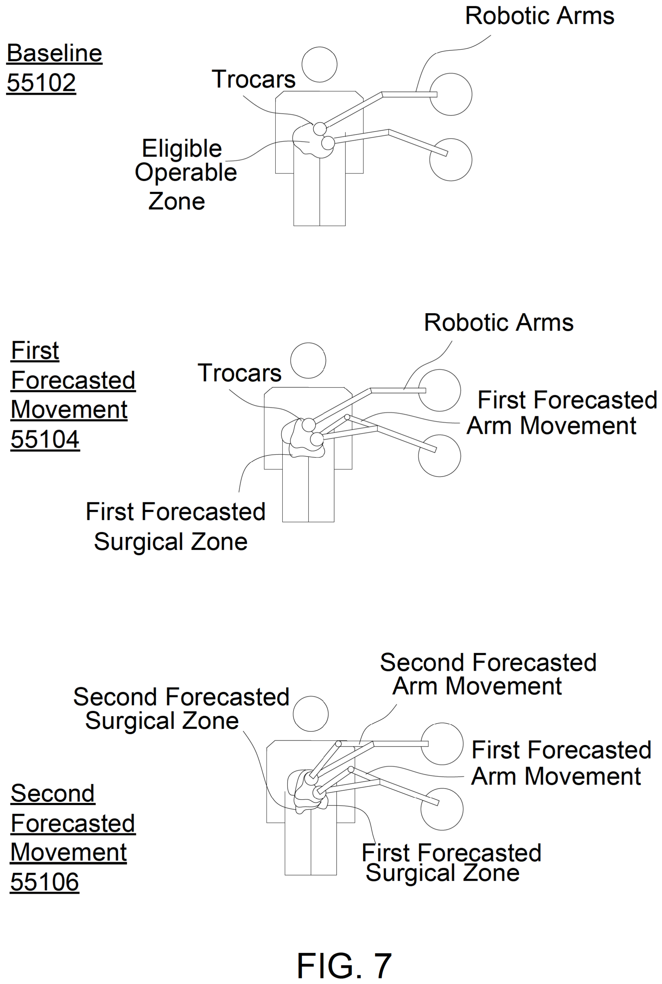

FIG. 7 illustrates an example display to a user of potential interactions between robotic arms during a surgical procedure. In a baseline position 55102 (shown in the top figure), the two robotic arms may be in an initial location. This initial location may be at a time just after the instruments attached to the ends of the arms were inserted into the patient via trocars. Based on this initial position, the system may determine an eligible operable zone. The eligible operable zone may indicate the areas in the patient's body that are accessible to the end effectors without moving arm joints external to the patient's body. The system may outline the eligible operable zone (e.g., within the surgical cavity) in a given orientation. The display may show locations of trocar ports through which the arms are inserted into the patient.

The surgeon may determine to move one of the arms using joints external to the patient (e.g., to access an area not in the eligible operable zone). The first forecasted move 55104 (e.g., first selected action, shown in the middle figure) illustrates an example movement of one of the arms. As shown, the system may update the displayed image to show an updated operable zone (e.g., a first forecasted surgical zone). The system may keep a depiction of the initial positioning of the arm. This may allow the surgeon to better visualize what the movement will look like. The surgeon may similarly move the other arm using joints external to the patient. The second forecasted move 55106 (e.g., second selected action, shown in the bottom figure) illustrates an example movement of the arm. As shown, the system may update the displayed image to show an updated operable zone (e.g., a second forecasted surgical zone). The system may keep a depiction of the initial positioning of the arm. These depictions may allow the surgeon to keep track of the positioning of the arms relative to each other, which may help the surgeon avoid collisions.

Robots may be equipped with means for determining the location of one arm relative to another in three-dimensional space. For example, another may monitor arms and provide relative measurements of one with respect to the other. For example, the system may determine the relative measurements through imaging of the OR. One or more cameras within the OR may be used to generate this information. The cameras may be separate from, or on the robot itself. The system may determine the relative measurements based on magnetic sensors, ultrasonic pinging, etc. This additional data feed may be used to determine the location of the devices relative to each other.

The hub and/or camera system may have to have stored parameter(s) related to the device(s) being tracked and/or capabilities of those device(s). Triangulation of the device position may be used to suggest device motions. For example, the system may use kinematics of the device(s) (e.g., robot arm(s)) and the patient to determine viable movement options. The kinematics may be derived (e.g., on the fly) using visualization. The range of motion (e.g., reach) and balance of the robot may be received from the robot or predetermined.

Indexing elements (e.g., fiducials) on the arms may enable a separate system to monitor the arms, (e.g., each of the segments of the arms). In some examples, electronic sensors may be used (e.g., rather than fiducials). The electronic sensors may emit a signal that is received by other sensors or a base station. For example, fiducials and/or electronic sensors may be placed at points 55108a-c (e.g., and/or other joints) in FIG. 5.

A virtual fence (e.g., a dynamic virtual fence) may be used to choose and reserve a spatial volume (e.g., adjacent the patient) during the time an HCP (e.g., a surgeon or physician's assistant) intends to occupy the space (e.g., to grant access to the patient without interfering with the flow of the procedure). The device may identify a reserved space occupied by a third surgical instrument. Determining the first candidate action and the second candidate action associated with a first surgical instrument may involve determining that the first candidate action and the second candidate action cause the first surgical instrument to remain outside of the reserved space.

The first candidate action may involve placing a port in a first port location on a patient. The second candidate action may involve placing the port in a second port location on the patient. The control signal may be configured to indicate one or more of: a first magnitude of access that a laparoscopic instrument will have to a surgical area if the first port location is used, a first number of orientation possibilities of the laparoscopic instrument if the first port location is used, a second magnitude of access that the laparoscopic instrument will have to a surgical area if the second port location is used, or a second number of orientation possibilities of the laparoscopic instrument if the second port location is used. The accessory trocar port location may be identified (e.g., during initial robotic port placement or at any other time during the procedure). A virtual fence defining a space the surgeon would occupy when using a device may be defined around the accessory trocar port.

The first surgical instrument may include a joint. The first candidate action may involve placing a port in a first port location on a patient. The second candidate action may involve placing the port in a second port location on the patient. The device may determine a first effect associated with the first port location and a second effect associated with the second port location based on at least one of: a position of a health care provider relative to the first surgical instrument, an articulation angle of the joint, a joint length of the joint, or a degree of freedom of the joint.

The first candidate action may involve a first placement of a base associated with the first surgical instrument, or a first movement of the first surgical instrument. The second candidate action may involve a second placement of the base associated with the first surgical instrument, or a second movement of the first surgical instrument.

The virtual fence may be dynamic in the sense that it can be turned on and off. The dynamic virtual fence may be turned on/off by the surgeon operating the robot. The dynamic virtual fence may be turned on/off by the robot itself (e.g., based on the next anticipated steps of the procedure).

The virtual fence location may be adjusted (e.g., based on the measured angle of the device that is observed to be passing through it by a laparoscopic camera). The laparoscopic camera may be oriented relative to the accessory trocar (e.g., during a setup procedure step) to register a common coordinate system and location. The virtual fence may adjust in size, shape, and/or location while on. If an accessory device is introduced through the trocar, the orientation of the device may be used to adjust the location/size/shape of the dynamic virtual fence. The surgeon operating the robot may adjust the location/size/shape of the dynamic virtual fence based on feedback from the user of the accessory trocar.

Anticipated procedure steps may be created using historical data from procedures with similar characteristics as the current one (e.g., patient factors, disease state, surgeon, device utilization, etc.). Anticipated procedure steps may be determined (e.g., directed) by the surgeon operating the robot.

The dynamic virtual fence may be slowly or instantaneously turned on. The robotic arms may adjust to this space to ensure that the fence is not violated by any portion of the arms. If the fence is turned on instantaneously, the robot may take a short period of time to reposition itself. This can be accomplished without changing the end location of devices attached to the robot arms (e.g., if the arms have sufficient degrees of freedom of motion).

If the robot anticipates that a procedure step is coming, the robot may make choices about how it moves its arms (e.g., to start to create the space prior to it being needed). This may result in less efficient movements leading up to the virtual fence being present. The movements may be accommodated through sufficient motor speed/direction choices, etc.

The device may receive user preference information and a patient position associated with the surgical procedure. The device may determine a surgical constraint based on at least one of the user preference information or the patient position. Selecting the action, from the first candidate action and the second candidate action, for the first surgical instrument to perform, may be based on the surgical constraint.

If the virtual fence has been created, the robot may respond to movement commands from the surgeon to move from location to location (e.g., using control algorithms that account for the constraint of not being able to violate the virtual fence).

For example, in thoracic resection procedures, there may be multiple (e.g., up to three distinct) tissues to staple (e.g., lung parenchyma, major pulmonary vessels, and major pulmonary airways). Specialty staplers may be used for the vessel and airway firings due to their unique access capabilities. Some robotic staplers may not have these access capabilities. The performance of some robotic staplers may be inferior to available handheld options. Handheld staplers may be used in robotic procedures. Space around the patient may be reserved for the HCP (e.g., a surgeon or physician's assistant) to reside to perform the firing (e.g., without interfering with or interference from the robot).

If the vessel and/or airway transection are complete, the reserved space may be removed (e.g., allowing the robot to return to an unconstrained state of movement based on the original algorithms).

The options of kinematic motion may be documented. Ambiguous displays may complicate the easy understanding of the choices the surgeon is making (e.g., in real-time or future steps).

The system may output a predictable display of sequential options. The choice the HCP is making may cause only arm-to-arm interaction outcomes. The choice may affect the orientation and position of the trocar and the internal surgical site. The choice may affect the remaining amount of head rotation or articulation angle of a robotic arm (e.g., beyond what is currently being used). The choice may affect the remaining degrees of freedom the end-effector has to get to locations beyond its current position.

The information being presented to the HCP may be relevant to the decisions being made or actions being taken at a point in time. The screen or display information may not (e.g., should not) include information that is no longer relevant, or a level of detail that is not appropriate.

The display options may help the HCP or operating room (OR) team understand the decisions the robot is making, and assist/control these decisions (e.g., as needed).

The system (e.g., robot) may display the current status of the surgical procedure and/or step. The system may display associated decision(s) and/or movement(s) being made by the system. The display elements may indicate the broader context of the decision(s) within the surgery (e.g., to give the HCP greater confidence and understanding of the decision(s) made by the system).

The system may nest choices within the context of a broader surgical step. For example, the system may provide a series of smaller steps as they relate to an overall larger surgical objective or goal.

The system may display collapsible views of non-active surgical steps.

To provide context, larger segments of the operation may be viewable and/or observable by the surgeon and/or surgical staff. To minimize the display of information (e.g., minimize information overload), non-active segments or broader steps may be collapsed.

The system may output an integrated display of a current autonomous step and upcoming non-autonomous step(s).

The system may provide context about upcoming steps that may require surgical guidance or action by the HCP (e.g., as opposed to autonomous action).

The system may indicate whether the decision is being made autonomously by the system or involves a human decision. The indication may be a light emitting diode (LED) display on the system, a screen display on the system (e.g., for staff), on a surgeon console, a light bar on the equipment/instrument, and/or the like.

The system may display forecasted decision(s). The system may provide an indication (e.g., in advance) of how a decision will be made or forecasted. The indication may be via a series of profiles, standard locations of equipment, imagery or text to provide the HCP or other users of the equipment the related information, and/or the like.

The system may display multiple step kinematic combinations (e.g., with a second variable, for example, probability of unintended interactions) to clearly articulate options to choose from or potential issues. The system may attempt to minimize arm interactions. The system may suggest arm placements (e.g., based on the patient size and orientation). The system may display the benefits/likelihood of arm interactions based on trocar/arm positions. For example, the system may display a topography map of likely interactions. The system may display a procedure map that highlights when and/or where arm interactions are expected to occur.

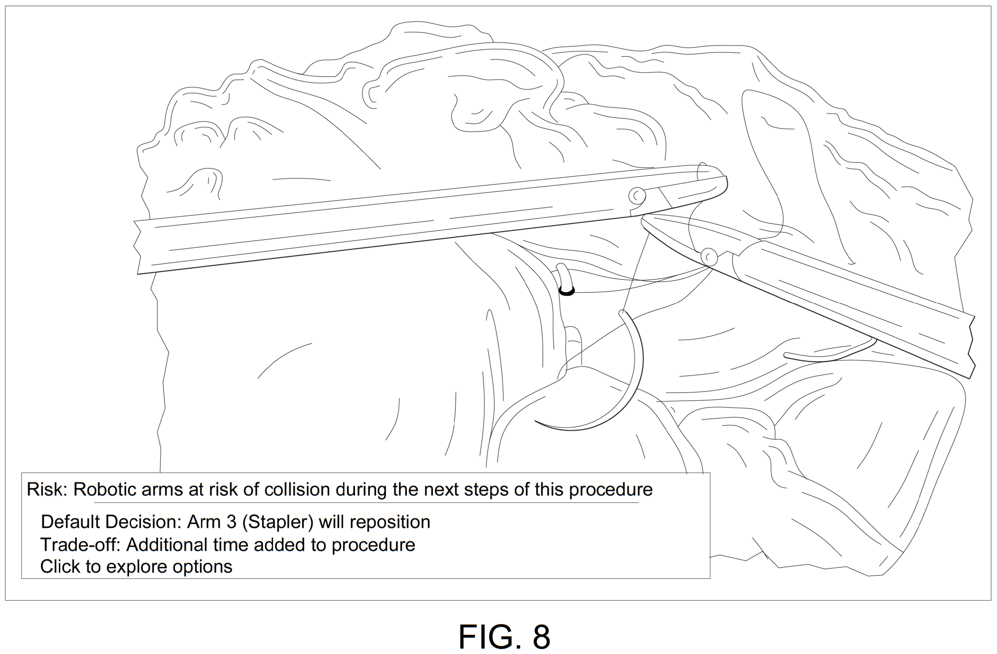

FIG. 8 illustrates an example display to a user of a decision to reposition a robotic arm to avoid a collision. As shown, the display may indicate that the robotic arms are at risk of colliding during one or more of the following steps of the procedure. The system may indicate that a default decision (e.g., if no user intervention is detected) may involve repositioning an arm (e.g., "Arm 3" with a stapler attached). The system may indicate that the default adjustment may increase the overall time of the procedure. The display may include a link that will allow the user to explore additional options (e.g., other than the default). The additional options may similarly include an indication of pros and/or cons associated with the options. For example, an additional option may be to reposition a different arm. The system may indicate that this option will likely cause another collision within the next few steps. These options and associated information may allow the surgeon to make informed decisions about how to proceed.

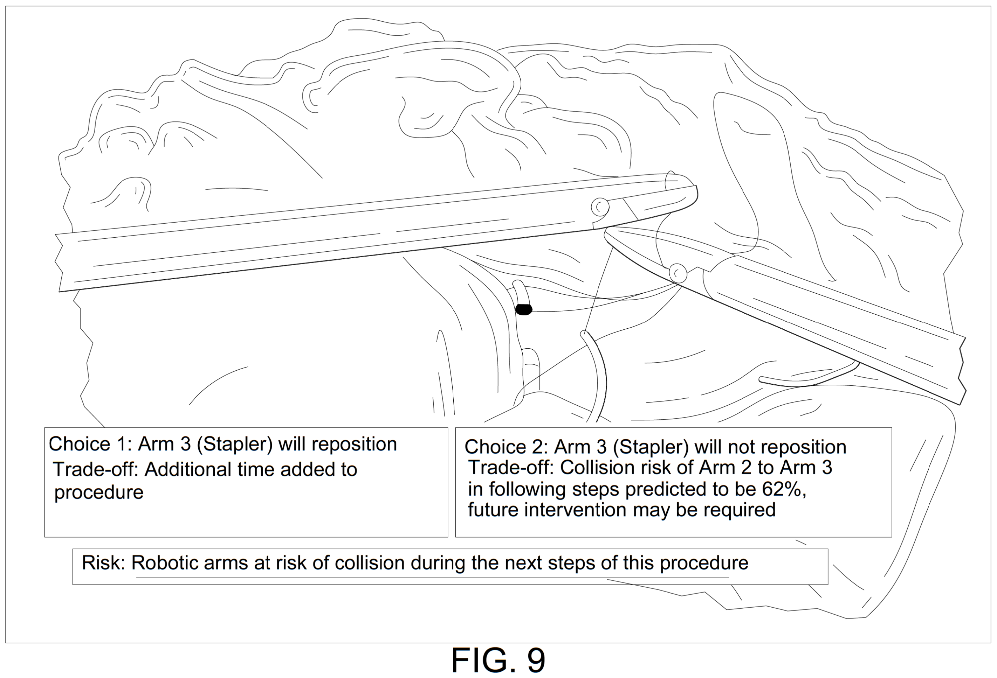

FIG. 9 illustrates an example display to a user of optional actions to avoid a collision. As shown, the display may indicate that the robotic arms are at risk of colliding during one or more of the following steps of the procedure. The system may indicate one or more options (e.g., choice 1 and choice 2 in FIG. 9). The system may indicate that one of the options is a preferred option. As shown, choice 1 may involve repositioning an arm (e.g., "Arm 3" with a stapler attached), which may increase the overall time of the procedure. In choice 2, Arm 3 may not be repositioned (e.g., another arm may be repositioned, or the surgeon may accept the risk of collision). The system may indicate that choice 2 is associated with a 62% collisions risk that will require future intervention. The surgeon may select one of the options or otherwise intervene to avoid the collision.

The system may propose change(s) throughout the procedure. For example, if a collision or interaction is about to occur, the system may determine how the user is notified/what is displayed. For example, the system may indicate options (e.g., whether the system should stop or change directions).

Collision avoidance may depend on the source of the instruction that will cause the collision. The system may determine its reaction to a potential collision based on the method by which it received the instruction that will cause the collision. For example, if the instruction is received (e.g., directly) from the surgeon, the system may allow the movement(s) to proceed (e.g., with a warning indication). An instruction that may (e.g., inadvertently) result in a collision that is created by the system (e.g., the system's control algorithms) may be stopped.

The system may indicate for the HCP to change a robot's position based on which a tool is attached. Coordinated colors and/or blinking on tools and a base may indicate a preferred location.

A system may have an LED at the base of the tool driver assembly (e.g., where it interconnects to the robotic system) and the housing or assembly portion that connects to the tool driver. The system may use color control of the LEDS to indicate the correct housing to the correct tool driver assembly.

For example, during initial setup for a surgical procedure with three tool housings, the tools may be connected to their appropriate housings. There may be three housings and three tools, each with their respective colors matching (e.g., Tool 1: Blue; Tool 2: Yellow; Tool 3: Red; Housing 1: Blue; Housing 2: Yellow; Housing 3: Red). As the procedure progresses, the system may indicate for the HCP to swap one or more tool location(s). For example, the following changes may be made. Tool 1: Blinking Red; Tool 2: Yellow (e.g., no change); Tool 3: Blinking Blue. The base or housing LEDs may not change colors (e.g., stay constant throughout a procedure). The blinking may indicate for the tools to be swapped to the identified new location.

The color of a housing indicator may be determined based on how it is identified in software. For example, in a multi-housing robotic system (e.g., Hugo robot), housing system #1 may (e.g., always) be mapped to 'Blue'; housing system #2 may (e.g., always) be mapped to 'Yellow'; and housing system #3 may (e.g., always) be mapped to 'Red.' If the mapping of these systems changes, the color identification may change as well.

Multi-factor information may be used to determine whether to switch a tool to another base. The system may display steps and/or instructions on the change to be made (e.g., with visible indications of the change to be made, for example, blinking LEDs on the tool and base).

The system may determine and display options from which the HCP may choose.

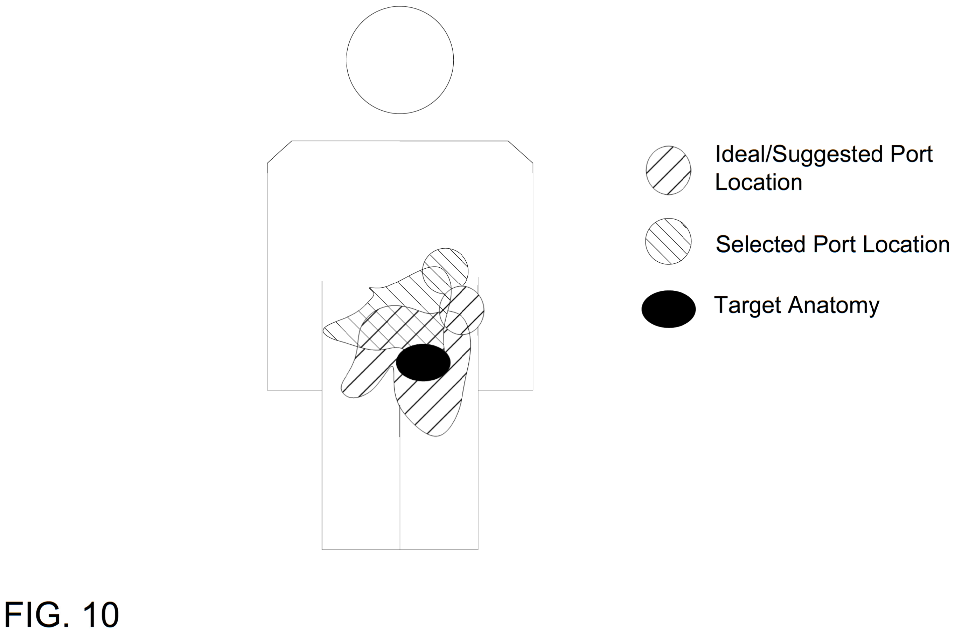

Instrument interactions may be affected by trocar placement and/or HCP position. The system may display the access port location and surgical site accessibility. FIG. 10 illustrates an example display to a user of the impact of potential port locations. As shown, the system may indicate a selected port location, an ideal/suggested port location, and a location of the target anatomy (e.g., to be reached via the port). The display may also show the areas that the end effector will be able to reach depending on which port is used. In the example in FIG. 10, the display allows the surgeon to visualize that the selected port location will not grant as much access to the target anatomy as the ideal/suggested port location would.

The port location of the trocars through the abdomen or thoracic walls may determine the angle of approach to the surgical site within the body. The configurations, articulation angle, joint length, degrees of freedom of the joints, and/or the rotational capabilities of the instruments may determine the viable approach angles and/or orientations of the end-effectors. The ability to visualize the interaction of the instrument choices and the access port locations may enable the surgeon to choose instruments that are capable of accessing the surgical site or what port locations to use (e.g., based on the choice of instruments).

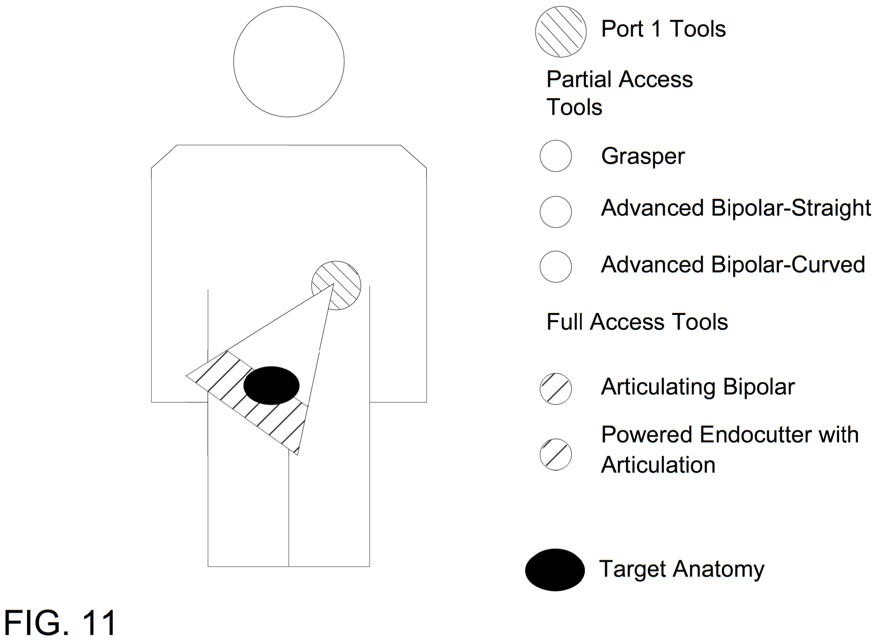

FIG. 11 illustrates an example display to a user of the access capabilities of surgical tools based on a port location. As shown, the system may indicate the areas in which different tools will be able to access via a first port location. The partial access tools (e.g., grasper, advanced bipolar-straight, and advanced bipolar-curved) may be able to reach the target anatomy but will not be able to access all of the target anatomy. The full access tools (e.g., articulating bipolar and a powered endocutter with articulation) may be able to access all of the target anatomy (and perhaps beyond the target anatomy).

A device may have actuation jaws with different configurations. For example, in an endocutter, one jaw may be used to house the cartridge and the staples (e.g., making it twice as large as the other jaw). In an endocutter, one of the two jaws may pivot, and the other jaw may be fixed to the shaft axis. In ultrasonic or bipolar advanced energy devices, the blade and the wave guide may be coupled to the shaft (e.g., or an aspect of the shaft). The clamp arm of the device may be moved relative to the shaft.

The system may determine an approach angle to the local surgical site. The system may determine an orientation (e.g., a preferred orientation) of the end-effector to the anatomic structures of the patient.

For example, in pulmonary artery/pulmonary vein (PA/PV) transection of the lung in a segmentectomy or lobectomy, the surgeon may prefer to have a non-moving jaw and the smallest profile jaw underneath the artery during introduction. This may reduce (e.g., minimize) the likelihood of tears or unnecessary tissue tension of the fragile artery. This may enable the least amount of skeletonization and dissection and may reduce (e.g., minimize) the likelihood of collateral damage. The access orientation of the shaft of the device may be used to position the end-effector perpendicular to the artery and vein (e.g., while avoiding other structures and having only a limited amount of access possibilities through the rib cage).

The system may show the magnitude of the difference of port placement on the instrument access and orientation possibilities (e.g., relative to the choice of differing instruments). The system may display a simulation of port placement relevant to toolset selection for a given placement.

The simulation may allow information to be presented to the surgeon. For example, the system may (e.g., dynamically) present information about different instruments for a given port placement. As the selected placement moves, the information regarding the utilizable tools may shift.

A composite rendering that highlights the differences of the ideal location (e.g., as opposed to the selected location) may be displayed.

The system may present information relative to target anatomy. For example, the system may display a simulation of port placements for a given surgical procedure and anticipated tools. The system may display optional location(s) of port placement based on anticipated variables (e.g., selected surgery and tools) and the impacts of using the location(s). The port placement(s) may reduce (e.g., minimize) robot arm interactions.

The location of the access ports and the instruments may interfere with the local imaging access options. The imaging systems may be affected by the local presence of metals, electromagnetic fields, capacitive coupling, and/or other energy-based interactions between the proximity and magnitude of the interactive effect. For example, the EM sensors (e.g., on the monarch flexible robotic scope) may be affected by the proximity and size of metal objects within the field (e.g., generated by the unit base station). The CT may reflect off of metal objects causing a glare occluding localized visualization. Impendence spectroscopy and/or other conductivity through the tissue may be affected by the presence of metal objects and saline near the imaging location. Capacitive coupling may occur if high levels of energy are moved down a shaft that is closely aligned to another conductive shaft. Capacitive coupling may result in parasitic drains on the sensing and/or unintended currents in the other devices (e.g., and potential burns within the patient).

The system may determine how to display complicated data (e.g., multiple data streams and context) so that the user (e.g., HCP) can make a decision (e.g., give input to the system).

The system may use an adaptive display of the data to visualize trends. The system may adapt how the data is presented to the surgeon (e.g., in real time), for example, based on the data stream(s).

The visualization/presentation of the data may be determined based on relative changes in parameters. For example, related features may be clustered to illustrate patterns around categorical variables. For example, vessel parameters (e.g., size, dissection, etc.) may be located adjacent to one another on the display.

The visualization/presentation of the data may be determined based on changes of parameters over time. For example, a mean and/or range dot plot may be used. An area chart may be used to display relative magnitude over time.

The visualization/presentation of the data may be determined based on interrelated parameters. For example, stacked summary bar charts may be used to illustrate multiple summed data streams (e.g., and the originating data trends). The data may be divided based on tissue type.

The visualized data may include ablation and/or drug delivery, tumor size (e.g., throughout removal), a probe coverage zone, a distance to critical structures, tumor density (e.g., susceptibility to treatment), surrounding tissue damage, and/or the like.

In the case of gallstone removal, the visualized data may include a percentage of successful identification of stones, stone size, percentage of natural passage, and/or the like. In another example, the system may determine that an object is a stone, but its location is somewhere that a stone would not be. In this case, the system may request doctor input to help identify the object. The system may explain why it is unsure of the object's identity (e.g., the location).

The data may be divided based on procedure type. For example, the data may show a percentage of likely robot arm collisions during the procedure.

The system may reduce (e.g., eliminate) noise via the diagramming. For example, rose diagramming may be used. The system may display co-morbidities related to a selected procedure approach.

The system may display breakout diagramming of multiple choices. For example, a sunburst pie chart may be used for relational display of magnitude and frequency of common data points. A percentage breakout of stacked bar charts may be used to display trends. For example, the Y axis may illustrate procedure steps, the X axis may represent time, and different colors may be used to differentiate between laparoscopic and endoscopic device data.

The visualization may be adapted based on smart device capabilities and/or magnitude of requested interpretation. An example miso algorithm may output data to a hub. The system may display tissue creep and/or force stabilization using a first diagramming type, and knife speed force implications may be displayed using a second diagramming type.

The system may output a haptic indication of lack of operation. The system may indicate a reason for the indication (e.g., full articulation giggle, pause giggle, unable to proceed, further required reaction giggle, etc.). The reason may be indicated in a non-visual manner (e.g., based on intensity, duration, and/or frequency of haptic feedback).

The system may adjust a graphical user interface (GUI) on a screen. The adjustment may improve visualization of the context of the data (e.g., the non-obvious relationship between data streams). For example, the magnitude of a displayed event may be adapted (e.g., based on a procedural step and/or the like). The scale of the displayed data may be adjusted. The scale may be adjusted based on a relationship between a localized event compared to an ongoing baseline.

The display may show an anomaly score of harmonic activations. This data may indicate how the device performance changes throughout the life of the device. The data may indicate if an activation seems abnormal. An anomaly score is an example metric that can be calculated by a smart system to characterize device behavior/performance. For example, an anomaly score may be produced by the smart system running an Isolation Forest ML algorithm. An anomaly score of zero or less may indicate that the data point is anomalous.

The device may determine a parameter change associated with the first effect and the second effect. The device may determine a data type associated with the parameter change. The device may determine a format of a graphical representation of the first effect and the second effect based on the data type. The device may generate the graphical representation based on the determined format. The control signal may be configured to instruct a display to display the generated graphical representation.

The device may determine the data type associated with the parameter change based on one or more of: an absolute change in a parameter over a period of time; a relative change in the parameter over the period of time; data trends of summed data streams of interrelated parameters; or an impact, of the first or second effect, to one or more surgical instruments.

A data point in the graphical representation of the data may represent an activation of the device. The graphical representation may be shown as the surgeon progresses through a procedure (e.g., so that the surgeon is able to determine if an activation was flagged as anomalous).

In an example, a patient may be placed into a hypothermic state. The rates of insufflation gases, smoke evacuation, suction irrigation, and/or the like may impact patient temperature. The system may display a rate of change of the patient's body temperature. The system may display data from other systems that might be driving the rate. For example, limits on patient heating and cooling may be relevant to the system's decision to change a rate of heating or cooling.

Although some aspects are described with respect to one or more robotic arms, a person of ordinary skill in the art will appreciate that these aspects may be used for any powered device (e.g., an articulable endocutter, etc.).

The following is a non-exhaustive list of embodiments that may or may not be claimed:
Example 1. A device comprising:
   a processor configured to:
   receive an indication of a surgical procedure that involves a first surgical instrument cooperating with a second surgical instrument, wherein the surgical procedure comprises a plurality of surgical steps;
   determine a first candidate action and a second candidate action associated with the first surgical instrument, wherein the first candidate action and the second candidate action allow the first surgical instrument to complete a first step of the plurality of the surgical steps;
   determine a first effect, caused by the first candidate action, on the second surgical instrument's ability to perform a second step of the plurality of surgical steps;
   determine a second effect, caused by the second candidate action, on the second surgical instrument's ability to perform the second step of the plurality of surgical steps;
   select, based on the first effect and the second effect, an action, from the first candidate action and the second candidate action, for the first surgical instrument to perform; and
   generate a control signal configured to indicate the selected action.
Example 2. The device of example 1, wherein the selected action is a first action, the control signal is a first control signal, the surgical procedure further comprises a third step, and the processor is further configured to:
   determine, based on the selected first action associated with the first surgical instrument, a third candidate action and a fourth candidate action associated with the second surgical instrument, wherein the third candidate action and the fourth candidate action allow the second surgical instrument to complete the second step;
   determine a third effect, caused by the selected candidate action associated with the first surgical instrument and the third candidate action, on a third surgical instrument's ability to perform the third step;
   determine a fourth effect, caused by the selected candidate action associated with the first surgical instrument and the fourth candidate action, on the third surgical instrument's ability to perform the third step;
   select, based on the third effect and the fourth effect, a second action, from the third candidate action and the fourth candidate action, for the second surgical instrument to perform; and
   generate a second control signal configured to indicate the second action.
Example 3. The device of example 1 or 2, wherein the processor is further configured to:
   determine a parameter change associated with the first effect and the second effect;
   determine a data type associated with the parameter change;
   determine a format of a graphical representation of the first effect and the second effect based on the data type; and
   generate the graphical representation based on the determined format, wherein the control signal is configured to instruct a display to display the generated graphical representation.
Example 4. The device of any one of examples 1-3, wherein the processor being configured to determine the data type associated with the parameter change comprises the processor being configured to determine the data type associated with the parameter change based on:
   an absolute change in a parameter over a period of time;
   a relative change in the parameter over the period of time;
   data trends of summed data streams of interrelated parameters; or
   an impact, of the first or second effect, on one or more surgical instruments.
Example 5. The device of any one of examples 1-4, wherein the processor is further configured to identify a reserved space occupied by a third surgical instrument, wherein the processor being configured to determine the first candidate action and the second candidate action associated with a first surgical instrument comprises the processor being configured to determine that the first candidate action and the second candidate action cause the first surgical instrument to remain outside of the reserved space.
Example 6. The device of any one of examples 1-5, wherein the first candidate action comprises placing a port in a first port location on a patient, the second candidate action comprises placing the port in a second port location on the patient, and the control signal is further configured to indicate one or more of: a first magnitude of access that a laparoscopic instrument will have to a surgical area if the first port location is used, a first number of orientation possibilities of the laparoscopic instrument if the first port location is used, a second magnitude of access that the laparoscopic instrument will have to a surgical area if the second port location is used, or a second number of orientation possibilities of the laparoscopic instrument if the second port location is used.
Example 7. The device of any one of examples 1-6, wherein the first surgical instrument comprises a joint, the first candidate action comprises placing a port in a first port location on a patient, the second candidate action comprises placing the port in a second port location on the patient, and the processor is further configured to determine a first effect associated with the first port location and a second effect associated with the second port location based on at least one of: a position of a health care provider relative to the first surgical instrument, an articulation angle of the joint, a joint length of the joint, or a degree of freedom of the joint.
Example 8. The device of any one of examples 1-7, wherein the processor is further configured to:
   receive user preference information and a patient position associated with the surgical procedure; and
   determine a surgical constraint based on at least one of the user preference information or the patient position, wherein the processor being configured to select the action, from the first candidate action and the second candidate action, for the first surgical instrument to perform comprises the processor being configured to select the action, from the first candidate action and the second candidate action, for the first surgical instrument to perform based on the surgical constraint.
Example 9. The device of any one of examples 1-8, wherein the first candidate action comprises a first placement of a base associated with the first surgical instrument, or a first movement of the first surgical instrument, and the second candidate action comprises a second placement of the base associated with the first surgical instrument, or a second movement of the first surgical instrument.
Example 10. A method comprising:
   receiving an indication of a surgical procedure that involves a first surgical instrument cooperating with a second surgical instrument, wherein the surgical procedure comprises a plurality of surgical steps;
   determining a first candidate action and a second candidate action associated with the first surgical instrument, wherein the first candidate action and the second candidate action allow the first surgical instrument to complete a first step of the plurality of the surgical steps;
   determining a first effect, caused by the first candidate action, on the second surgical instrument's ability to perform a second step of the plurality of surgical steps;
   determining a second effect, caused by the second candidate action, on the second surgical instrument's ability to perform the second step of the plurality of surgical steps;
   selecting, based on the first effect and the second effect, an action, from the first candidate action and the second candidate action, for the first surgical instrument to perform; and
   generating a control signal configured to indicate the selected action.
Example 11. The method of example 10, wherein the selected action is a first action, the control signal is a first control signal, the surgical procedure further comprises a third step, and the method further comprises:
   determining, based on the selected first action associated with the first surgical instrument, a third candidate action and a fourth candidate action associated with the second surgical instrument, wherein the third candidate action and the fourth candidate action allow the second surgical instrument to complete the second step;
   determining a third effect, caused by the selected candidate action associated with the first surgical instrument and the third candidate action, on a third surgical instrument's ability to perform the third step;
   determining a fourth effect, caused by the selected candidate action associated with the first surgical instrument and the fourth candidate action, on the third surgical instrument's ability to perform the third step;
   selecting, based on the third effect and the fourth effect, a second action, from the third candidate action and the fourth candidate action, for the second surgical instrument to perform; and
   generating a second control signal configured to indicate the second action.
Example 12. The method of example 10 or 11, wherein the method further comprises:
   determining a parameter change associated with the first effect and the second effect;
   determining a data type associated with the parameter change;
   determining a format of a graphical representation of the first effect and the second effect based on the data type; and
   generating the graphical representation based on the determined format, wherein the control signal is configured to instruct a display to display the generated graphical representation.
Example 13. The method of any one of examples 10-12, wherein determining the data type associated with the parameter change comprises determining the data type associated with the parameter change based on:
   an absolute change in a parameter over a period of time;
   a relative change in the parameter over the period of time;
   data trends of summed data streams of interrelated parameters; or
   an impact, of the first or second effect, on one or more surgical instruments.
Example 14. The method of any one of examples 10-13, wherein the method further comprises identifying a reserved space occupied by a third surgical instrument, wherein determining the first candidate action and the second candidate action associated with a first surgical instrument comprises determining that the first candidate action and the second candidate action cause the first surgical instrument to remain outside of the reserved space.
Example 15. The method of any one of examples 10-14, wherein the first candidate action comprises placing a port in a first port location on a patient, the second candidate action comprises placing the port in a second port location on the patient, and the control signal is further configured to indicate one or more of: a first magnitude of access that a laparoscopic instrument will have to a surgical area if the first port location is used, a first number of orientation possibilities of the laparoscopic instrument if the first port location is used, a second magnitude of access that the laparoscopic instrument will have to a surgical area if the second port location is used, or a second number of orientation possibilities of the laparoscopic instrument if the second port location is used.
Example 16. The method of any one of examples 10-15, wherein the first surgical instrument comprises a joint, the first candidate action comprises placing a port in a first port location on a patient, the second candidate action comprises placing the port in a second port location on the patient, and the method further comprises determining a first effect associated with the first port location and a second effect associated with the second port location based on at least one of: a position of a health care provider relative to the first surgical instrument, an articulation angle of the joint, a joint length of the joint, or a degree of freedom of the joint.
Example 17. The method of any one of examples 10-16, wherein the method further comprises:
   receiving user preference information and a patient position associated with the surgical procedure; and
   determining a surgical constraint based on at least one of the user preference information or the patient position, wherein selecting the action, from the first candidate action and the second candidate action, for the first surgical instrument to perform, is further based on the surgical constraint.
Example 18. The method of any one of examples 10-17, wherein the first candidate action comprises a first placement of a base associated with the first surgical instrument, or a first movement of the first surgical instrument, and the second candidate action comprises a second placement of the base associated with the first surgical instrument, or a second movement of the first surgical instrument.

Although features and elements are described above in particular combinations, one of ordinary skill in the art will appreciate that each feature or element can be used alone or in any combination with the other features and elements. In addition, the methods described herein may be implemented in a computer program, software, or firmware incorporated in a computer-readable medium for execution by a computer or processor. Examples of computer-readable media include electronic signals (transmitted over wired or wireless connections) and computer-readable storage media. Examples of computer-readable storage media include, but are not limited to, a read only memory (ROM), a random access memory (RAM), a register, cache memory, semiconductor memory devices, magnetic media such as internal hard disks and removable disks, magnetooptical media, and optical media such as CD-ROM disks, and digital versatile disks (DVDs). A processor in association with software may be used to implement a radio frequency transceiver for use in a WTRU, UE, terminal, base station, RNC, or any host computer.

## Claims

1. A device comprising a processor configured to:
receive an indication of a surgical procedure that involves a first surgical instrument cooperating with a second surgical instrument, wherein the surgical procedure comprises a plurality of surgical steps;
determine a first candidate action and a second candidate action associated with the first surgical instrument, wherein the first candidate action and the second candidate action allow the first surgical instrument to complete a first step of the plurality of the surgical steps;
determine a first effect, caused by the first candidate action, on the second surgical instrument's ability to perform a second step of the plurality of surgical steps;
determine a second effect, caused by the second candidate action, on the second surgical instrument's ability to perform the second step of the plurality of surgical steps;
select, based on the first effect and the second effect, an action, from the first candidate action and the second candidate action, for the first surgical instrument to perform; and
generate a control signal configured to indicate the selected action.

2. The device of claim 1, wherein the selected action is a first action, the control signal is a first control signal, the surgical procedure further comprises a third step, and the processor is further configured to:
determine, based on the selected first action associated with the first surgical instrument, a third candidate action and a fourth candidate action associated with the second surgical instrument, wherein the third candidate action and the fourth candidate action allow the second surgical instrument to complete the second step;
determine a third effect, caused by the selected candidate action associated with the first surgical instrument and the third candidate action, on a third surgical instrument's ability to perform the third step;
determine a fourth effect, caused by the selected candidate action associated with the first surgical instrument and the fourth candidate action, on the third surgical instrument's ability to perform the third step;
select, based on the third effect and the fourth effect, a second action, from the third candidate action and the fourth candidate action, for the second surgical instrument to perform; and
generate a second control signal configured to indicate the second action.

3. The device of claim 1 or claim 2, wherein the processor is further configured to:
determine a parameter change associated with the first effect and the second effect;
determine a data type associated with the parameter change;
determine a format of a graphical representation of the first effect and the second effect based on the data type; and
generate the graphical representation based on the determined format, wherein the control signal is configured to instruct a display to display the generated graphical representation, optionally wherein the processor is configured to determine the data type associated with the parameter change based on:
an absolute change in a parameter over a period of time;
a relative change in the parameter over the period of time;
data trends of summed data streams of interrelated parameters; or
an impact, of the first or second effect, on one or more surgical instruments.

4. The device of any one of the preceding claims, wherein the processor is further configured to identify a reserved space occupied by a third surgical instrument, wherein the processor being configured to determine the first candidate action and the second candidate action associated with a first surgical instrument comprises the processor being configured to determine that the first candidate action and the second candidate action cause the first surgical instrument to remain outside of the reserved space.

5. The device of any one of the preceding claims, wherein the first surgical instrument comprises a joint, the first candidate action comprises placing a port in a first port location on a patient, the second candidate action comprises placing the port in a second port location on the patient, and the processor is further configured to determine a first effect associated with the first port location and a second effect associated with the second port location based on at least one of: a position of a health care provider relative to the first surgical instrument, an articulation angle of the joint, a joint length of the joint, or a degree of freedom of the joint.

6. The device of any one of the preceding claims, wherein the processor is further configured to:
receive user preference information and a patient position associated with the surgical procedure; and
determine a surgical constraint based on at least one of the user preference information or the patient position, wherein the processor being configured to select the action, from the first candidate action and the second candidate action, for the first surgical instrument to perform comprises the processor being configured to select the action, from the first candidate action and the second candidate action, for the first surgical instrument to perform based on the surgical constraint.

7. A method performed by a processor, the method comprising:
receiving an indication of a surgical procedure that involves a first surgical instrument cooperating with a second surgical instrument, wherein the surgical procedure comprises a plurality of surgical steps;
determining a first candidate action and a second candidate action associated with the first surgical instrument, wherein the first candidate action and the second candidate action allow the first surgical instrument to complete a first step of the plurality of the surgical steps;
determining a first effect, caused by the first candidate action, on the second surgical instrument's ability to perform a second step of the plurality of surgical steps;
determining a second effect, caused by the second candidate action, on the second surgical instrument's ability to perform the second step of the plurality of surgical steps;
selecting, based on the first effect and the second effect, an action, from the first candidate action and the second candidate action, for the first surgical instrument to perform; and
generating a control signal configured to indicate the selected action.

8. The method of claim 7, wherein the selected action is a first action, the control signal is a first control signal, the surgical procedure further comprises a third step, and the method further comprises:
determining, based on the selected first action associated with the first surgical instrument, a third candidate action and a fourth candidate action associated with the second surgical instrument, wherein the third candidate action and the fourth candidate action allow the second surgical instrument to complete the second step;
determining a third effect, caused by the selected candidate action associated with the first surgical instrument and the third candidate action, on a third surgical instrument's ability to perform the third step;
determining a fourth effect, caused by the selected candidate action associated with the first surgical instrument and the fourth candidate action, on the third surgical instrument's ability to perform the third step;
selecting, based on the third effect and the fourth effect, a second action, from the third candidate action and the fourth candidate action, for the second surgical instrument to perform; and
generating a second control signal configured to indicate the second action.

9. The method of claim 7 or claim 8, wherein the method further comprises:
determining a parameter change associated with the first effect and the second effect;
determining a data type associated with the parameter change;
determining a format of a graphical representation of the first effect and the second effect based on the data type; and
generating the graphical representation based on the determined format, wherein the control signal is configured to instruct a display to display the generated graphical representation, optionally wherein determining the data type associated with the parameter change is based on:
an absolute change in a parameter over a period of time;
a relative change in the parameter over the period of time;
data trends of summed data streams of interrelated parameters; or
an impact, of the first or second effect, on one or more surgical instruments.

10. The method of claim any one of claims 7 to 9, wherein the method further comprises identifying a reserved space occupied by a third surgical instrument, wherein determining the first candidate action and the second candidate action associated with a first surgical instrument comprises determining that the first candidate action and the second candidate action cause the first surgical instrument to remain outside of the reserved space.

11. The device of any one of claims 1 to 6 or the method of any one of claims 7 to 10, wherein the first candidate action comprises placing a port in a first port location on a patient, the second candidate action comprises placing the port in a second port location on the patient, and the control signal is further configured to indicate one or more of: a first magnitude of access that a laparoscopic instrument will have to a surgical area if the first port location is used, a first number of orientation possibilities of the laparoscopic instrument if the first port location is used, a second magnitude of access that the laparoscopic instrument will have to a surgical area if the second port location is used, or a second number of orientation possibilities of the laparoscopic instrument if the second port location is used.

12. The method of any one of claims 7 to 11, wherein the first surgical instrument comprises a joint, the first candidate action comprises placing a port in a first port location on a patient, the second candidate action comprises placing the port in a second port location on the patient, and the method further comprises determining a first effect associated with the first port location and a second effect associated with the second port location based on at least one of: a position of a health care provider relative to the first surgical instrument, an articulation angle of the joint, a joint length of the joint, or a degree of freedom of the joint.

13. The method of any one of claims 7 to 12, wherein the method further comprises:
receiving user preference information and a patient position associated with the surgical procedure; and
determining a surgical constraint based on at least one of the user preference information or the patient position, wherein selecting the action, from the first candidate action and the second candidate action, for the first surgical instrument to perform, is further based on the surgical constraint.

14. The device of any one of claims 1 to 6 or the method of any one of claims 7 to 13, wherein the first candidate action comprises a first placement of a base associated with the first surgical instrument, or a first movement of the first surgical instrument, and the second candidate action comprises a second placement of the base associated with the first surgical instrument, or a second movement of the first surgical instrument.

15. A computer-readable medium comprising instructions which, when executed by a processor, cause the processor to carry out the method of any one of claims 7 to 14.
